# EUROPEAN PATENT SPECIFICATION

(11) **EP 3 377 651 B1**
(45) Date of publication and mention of the grant of the patent: **11.09.2019**
(21) Application number: 16822268.5
(22) Date of filing: 16.11.2016
(51) Int. Cl.: C12Q 1/68

(54) **A METHOD AND A KIT FOR NUCLEIC ACID DETECTION**
VERFAHREN UND KIT ZUR NUKLEINSÄUREDETEKTION
MÉTHODE ET TROUSSE DE DÉTECTION D'ACIDES NUCLÉIQUES

(30) Priority: 18.11.2015 IT UB20155706
(43) Date of publication of application: 26.09.2018
(73) Proprietor: Fondazione Istituto Italiano di Tecnologia, 16163 Genova (IT)
(72) Inventor: POMPA, Pier Paolo, 73100 Lecce (IT); VALENTINI, Paola, 16131 Genova (IT)
(74) Representative: Comoglio, Elena
(86) International application number: PCT/IB2016/056879
(87) International publication number: WO 2017/085627

(56) References cited:
- WO-A2-03/054214
- WO-A2-2006/088910
- WO-A2-2011/063388
- US-A1- 2011 124 518
- ZHANG DAVID YU: "Cooperative hybridization of oligonucleotides.", JOURNAL OF THE AMERICAN CHEMICAL SOCIETY 2 FEB 2011, vol. 133, no. 4, 2 February 2011 (2011-02-02), pages 1077-1086, XP002757318, ISSN: 1520-5126
- ALBAGLI D ET AL: "Chemical amplification (CHAMP) by a continuous, self-replicating oligonucleotide-based system", JOURNAL OF THE AMERICAN CHEMICAL SOCIETY, AMERICAN CHEMICAL SOCIETY, US, vol. 121, 1 January 1999 (1999-01-01), pages 6954-6955, XP002232541, ISSN: 0002-7863, DOI: 10.1021/JA990434Y
- NAKAYAMA SHIZUKA ET AL: "Junction probes - sequence specific detection of nucleic acids via template enhanced hybridization processes.", JOURNAL OF THE AMERICAN CHEMICAL SOCIETY 24 SEP 2008, vol. 130, no. 38, 24 September 2008 (2008-09-24), pages 12560-12561, XP002757319, ISSN: 1520-5126

## Description

The present invention relates to a new method for the detection of target nucleic acids, and to a related kit.

The detection of DNA and RNA target sequences is of the utmost importance in molecular diagnostics and, in this sector the amplification of nucleic acid sequences, which is necessary in order to identify the target, which usually is diluted in a mixture of interfering genetic material, is a general issue.

In standard laboratory tests, the amplification of nucleic acids is generally carried out by means of the polymerase chain reaction (PCR) or through other PCR-based techniques, such as for example real-time PCR (RT-PCR) or digital PCR. These techniques require cyclic variation of the reaction temperature, the use of dedicated equipment, the use of trained staff and, in some cases, the use of expensive reagents.

The need to allow molecular assays based on the detection of nucleic acids to be used outside of specialized laboratories, in order to provide for diagnostic assays that can be carried out in the vicinity of the patient's point of care and assistance (point-of-care diagnostic tests), has led to the development of numerous isothermal amplification techniques, which do not require cyclic variation of the temperature and which, therefore, allow for the use of more simple equipment. However, many of these isothermal techniques have technical drawbacks that so far have not allowed them to be actually applied extensively in commercial molecular assays. A non-exhaustive list of such drawbacks is provided below:
- The need for complex mixtures of enzymes/proteins, such as for example in the Helicase Dependent Amplification (HDA) (Vincent, M., Y. Xu, and H. Kong, Helicase-dependent isothermal DNA amplification. EMBO Rep, 2004. 5(8): p. 795-800), in the Recombinase Polymerase Amplification (RPA) (Piepenburg, O., et al., DNA detection using recombination proteins. PLoS Biol, 2006. 4(7): p. e204) and in the Nucleic Acid Sequence Based Amplification (NASBA) (Compton, J., Nucleic acid sequence-based amplification. Nature, 1991. 350(6313): p. 91-2). This requirement in turn entails high costs and careful processing and storage of reagents.

- The need for some post-processing steps in order to obtain the actual target, as in the case of the Rolling Circle Amplification (RCA) (Liu, D., et al., Rolling Circle DNA Synthesis: Small Circular Oligonucleotides as Efficient Templates for DNA Polymerases. J Am Chem Soc, 1996. 118(7): p. 1587-1594), which requires cleavage with restriction endonucleases.
- Non-specific amplification issues, which are typical of techniques based on the combination of a polymerase and an endonuclease designed to cut a single strand (nicking), as the Exponential Amplification Reaction (EXPAR) (Van Ness, J., L.K. Van Ness, and D.J. Galas, Isothermal reactions for the amplification of oligonucleotides. Proc Natl Acad Sci U S A, 2003. 100(8): p. 4504-9) or the Primer Generation-Rolling Circle Amplification (PG-RCA) (Murakami, T., J. Sumaoka, and M. Komiyama, Sensitive isothermal detection of nucleic-acid sequence by primer generation-rolling circle amplification. Nucleic Acids Res, 2009. 37(3): p. e19).
- The design of a complex mixture of primers, as in the case of the Loop-Mediated Isothermal Amplification (LAMP) (Notomi, T., et al., Loop-mediated isothermal amplification of DNA. Nucleic Acids Res, 2000. 28(12): p. E63) and of the Cross-Priming Amplification (CPA) (EP 2382330 A1).
- The complexity of the amplification product, a disadvantage shared by many techniques such as LAMP, CPA, RCA, which in turn limits the number of applicable procedures for detection of the outcome.

Some of the more simple isothermal procedures still require the use of two enzymes. For instance, the Strand Displacement Amplification (SDA) (Walker, G.T., et al., Isothermal in vitro amplification of DNA by a restriction enzyme/DNA polymerase system. Proc Natl Acad Sci USA, 1992. 89(1): p. 392-6) and the Nicking and Extension Amplification Reaction (NEAR) (US 2009/0081670 A1) both require a polymerase as well as a nicking endonuclease, while the Ramification Amplification (RAM) requires a ligase and a polymerase (Yao, B., et al., Quantitative analysis of zeptomole microRNAs based on isothermal ramification amplification. RNA, 2009. 15(9): p. 1787-94).

In order to overcome these and other drawbacks of the prior art, the present invention provides a new technique for the detection of nucleic acids, designated as "Cooperative Hybridization Mediated Isothermal Amplification of Nucleic Acids" (CHAMP). This is a simple procedure that can be carried out in a single test tube and that allows for the amplification of a nucleic acid sequence, for example a target DNA or a reporter sequence, by using a single enzyme, in particular an endonuclease designed to cut a single strand (nicking). This is obtained by means of a smart design of probes that exploit the cooperative hybridization of oligonucleotides (also designated as "base-stacking stabilization"). This phenomenon refers to the stabilization of the hybridization of a short oligonucleotide to a DNA template strand, due to base-stacking interactions of the terminal base of the first oligonucleotide with that of a second adjacent oligonucleotide that hybridizes in tandem with the same template strand (Khrapko, K.R., et al., A method for DNA sequencing by hybridization with oligonucleotide matrix. DNA Seq, 1991. 1(6): p. 375-88; Zhang, D.Y., Cooperative hybridization of oligonucleotides. J Am Chem Soc, 2011. 133(4): p. 1077-86; Lane, M.J., et al., The thermodynamic advantage of DNA oligonucleotide 'stacking hybridization' reactions: energetics of a DNA nick. Nucleic Acids Res, 1997. 25(3): p. 611-7; Broude, N.E., et al., Enhanced DNA sequencing by hybridization. Proc Natl Acad Sci U S A, 1994. 91(8): p. 3072-6; Fu, D.J., et al., A DNA sequencing strategy that requires only five bases of known terminal sequence for priming. Proc Natl Acad Sci USA, 1995. 92(22): p. 10162-6; Valentini, P., et al., Gold-Nanoparticle-Based Colorimetric Discrimination of Cancer-Related Point Mutations with Picomolar Sensitivity. ACS Nano, 2013. 7 (6): p. 5530-8).

The amplification technique which forms the object of the present invention has the advantage of being extremely simple and universal. The nicking site is provided by the probe, which determines the absence of any limitation with respect to the choice of the target, which may have any sequence. In addition, CHAMP is not affected by any of the aforementioned problems of the prior art. A unique and distinctive feature of CHAMP is that of being a polymerase-free reaction.

The nucleic acid detection technique which forms the object of the present invention is provided in two alternative embodiments. The first provides a linear amplification of a DNA reporter sequence. This first embodiment is designated as "linear CHAMP". The second embodiment provides an exponential amplification of the target sequence. It is designated as "exponential CHAMP".

Both embodiments can be coupled with various subsequent detection techniques; for example, in a non-limiting example, with a fluorescence detection for a real-time reading of the outcome ("*real-time readout*") or with a colorimetric detection, for example based on gold nanoparticles.

Therefore, a first object of the present invention is a method of detecting a target nucleic acid sequence (T) in a nucleic acid sample, the method comprising the steps of:
a) adding to the nucleic acid sample a reaction mixture comprising:
   i) an endonuclease enzyme;
   ii) an excess of a long probe (LP) consisting of a single-stranded oligonucleotide consisting of:
      - a 3' reporter sequence (R),
      - an endonuclease enzyme recognition site (NE), and
      - a 5' ΔcT sequence complementary to a portion of the target nucleic acid sequence to be detected; and
   iii) an excess of a short probe (SP) consisting of a single-stranded oligonucleotide consisting of:
      - a 5' ΔcR sequence complementary to a portion of the reporter sequence, and
      - a 3' cNE sequence complementary to the restriction enzyme recognition site,
   wherein both the 5' ΔcT sequence and the 5' ΔcR sequence are sufficiently short so that hybridization of the short probe to the long probe does not occur in the absence of the target nucleic acid sequence, and
   wherein the reaction mixture does not contain a polymerase enzyme;
b) allowing the reaction mixture to react with the nucleic acid sample at a temperature at which the endonuclease is active, whereby, if the target nucleic acid sequence is present in the nucleic acid sample, a double-stranded endonuclease enzyme recognition site is formed by cooperative hybridization of the short probe and the target nucleic acid sequence to the long probe, and the double-stranded endonuclease enzyme recognition site is then nicked or cleaved by the endonuclease, whereby a free reporter sequence is released from the long probe; and
c) detecting the free reporter sequence as an indication of the presence of the target nucleic acid sequence in the nucleic acid sample.

The detection method described above corresponds to the nucleic acid detection technique designated as "linear CHAMP".

A second object of the present invention is a kit for carrying out the linear CHAMP, the kit comprising:
i) an endonuclease enzyme;
ii) a long probe (LP) consisting of a single-stranded oligonucleotide consisting of:
   - a 3' reporter sequence (R),
   - an endonuclease recognition site (NE), and
   - a 5' ΔcT sequence complementary to a portion of the target nucleic acid sequence to be detected; and
iii) a short probe (SP) consisting of a single-stranded oligonucleotide consisting of:
   - a 5' ΔcR sequence complementary to a portion of the reporter sequence, and
   - a 3' cNE sequence complementary to the restriction enzyme recognition site,
wherein both the 5' ΔcT sequence and the 5' ΔcR sequence are sufficiently short so that hybridization of the short probe to the long probe does not occur in the absence of the target nucleic acid sequence,
and wherein the kit does not contain a polymerase enzyme.

A third object of the present invention is a method of detecting a target nucleic acid sequence (T) in a nucleic acid sample, the method comprising the steps of:
a) adding to the nucleic acid sample a reaction mixture comprising:
   i) an endonuclease enzyme;
   ii) an excess of a long probe (LP-exp) consisting of a single-stranded oligonucleotide consisting of:
      - a 3' sequence consisting of the target nucleic acid sequence (T) or a portion thereof (ΔT),
      - an endonuclease recognition site (NE), and
      - a 5' Δ1cT sequence which is complementary to a first portion of the nucleic acid sequence to be detected;
   iii) an excess of a first short probe (SP1-exp) consisting of a single-stranded oligonucleotide consisting of:
      - a 5' Δ2cT sequence which is complementary to a second portion of the nucleic acid sequence to be detected, and
      - a 3' Δ1cNE sequence which is complementary to a first portion of the endonuclease recognition site; and
   iv) an excess of a second short probe (SP2-exp) consisting of a single-stranded oligonucleotide consisting of:
      - a Δ2cNE sequence which is complementary to a second portion of the endonuclease recognition site, wherein the Δ1cNE sequence of the first short probe together with the Δ2cNE sequence of the second short probe make up the complement of the whole endonuclease recognition site,
   wherein the Δ2cNE sequence is sufficiently short that hybridization of the second short probe to the long probe does not occur in the absence of the target nucleic acid sequence,
   and wherein the reaction mixture does not comprise a polymerase enzyme;
b) allowing the reaction mixture to react with the nucleic acid sample at a temperature at which the endonuclease is active, whereby, if the target nucleic acid sequence is present in the nucleic acid sample, a double-stranded endonuclease recognition site is formed by cooperative hybridization of the first short probe, second short probe and target nucleic acid sequence to the long probe, and the double-stranded endonuclease recognition site is nicked or cleaved by the endonuclease, whereby a free target nucleic acid sequence or a portion thereof is released from the long probe; and
c) detecting the free target nucleic acid sequence or a portion thereof as an indication of the presence of the target nucleic acid sequence in the nucleic acid sample.

The detection method described above corresponds to the nucleic acid detection technique designated as "exponential CHAMP".

A fourth object of the present invention is a kit for carrying out the exponential CHAMP, the kit comprising:
i) an endonuclease enzyme;
ii) a long probe (LP-exp) consisting of a single-stranded oligonucleotide consisting of:
   - a 3' sequence consisting of the target nucleic acid sequence or a portion thereof (ΔT),
   - an endonuclease recognition site (NE), and
   - a 5' Δ1cT sequence which is complementary to a first portion of the target nucleic acid sequence to be detected;
iii) a first short probe (SP1-exp) consisting of a single-stranded oligonucleotide consisting of:
   - a 5' Δ2cT sequence which is complementary to a second portion of the nucleic acid sequence to be detected, and
   - a 3' Δ1cNE sequence which is complementary to a first portion of the endonuclease recognition site; and
iv) a second short probe (SP2-exp) consisting of a single-stranded oligonucleotide consisting of:
   - a Δ2cNE sequence which is complementary to a second portion of the endonuclease recognition site, wherein the Δ1cNE sequence of the first short probe together with the Δ2cNE sequence of the second short probe make up the complement of the whole endonuclease recognition site,
wherein the Δ2cNE sequence is sufficiently short that hybridization of the second short probe to the long probe does not occur in the absence of the target nucleic acid sequence,
and wherein the kit does not comprise a polymerase enzyme.

Further features and advantages of the invention will be apparent from the detailed description which follows, given purely by way of non-limiting example, with reference to the accompanying drawings, in which:
Figures 1 and 2 schematically illustrate the steps of the linear CHAMP method according to the invention;
Figures 3 and 4 schematically illustrate the steps of the exponential CHAMP method according to the invention;
Figures 5 and 6 schematically illustrate an embodiment of the linear CHAMP which entails the use of an additional probe, designated as "displacing probe".

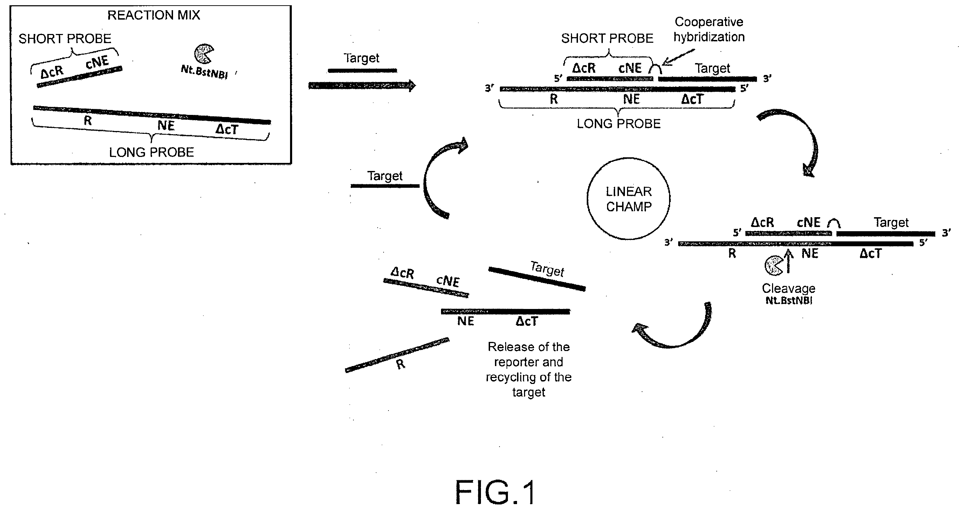

Figure 1 shows the linear version of CHAMP which forms the object of the present invention. It uses a synthetic single-stranded nucleic acid template (e.g. ssDNA) designated as long probe (LP) comprising a 3' reporter sequence (R), a nicking endonuclease recognition site (NE) and a 5' sequence which is partially complementary to that of the target of interest (ΔcT). This template is in solution at a very high concentration, together with a shorter single-stranded nucleic acid (e.g. ssDNA), designated as short probe (SP), which comprises a 5' sequence (ΔcR) that is partially complementary to the reporter sequence and a 3' sequence (cNE) that is complementary to the NE site. Therefore, the sequence of the short probe is complementary to a portion of the sequence of the long probe.

Preferably, the 5' ΔcT sequence has a length comprised between 10 and 20 nucleotides.

Preferably, the 5' ΔcR sequence has a length comprised between 0 and 10 nucleotides. In fact, in the case where an endonuclease having a low operating temperature, such as for example 37°C, were selected, the length of the short probe should be reduced to a minimum to avoid hybridization in the absence of the target. The shortest usable sequence for the long probe must include the entire endonuclease recognition site, but not necessarily part of the reporter sequence, and so, in this case, ΔcR may not be present.

In addition to the short probe and the long probe, the reaction mixture also comprises a nicking endonuclease enzyme, which nicks a single strand. The preferred nicking endonuclease is Nt.BstNBI. The reaction mixture also contains a buffer that provides the appropriate conditions required for the enzymatic activity.

The reaction is carried out at a constant temperature at which the enzyme used is active, for example comprised between 50°C and 70°C, preferably at about 55°C. The reaction temperature is selected based on the optimum temperature for the specific enzyme used. At this temperature, the short probe is not able to hybridize to the long probe, whereby the two single-stranded probes are in solution as two separate strands. However, in the presence of the target (T), the hybridization of the short probe and the target with the complementary portion of the long probe becomes possible, thanks to the base-stacking interactions between the 3'-terminus base of the short probe and the 5'-terminus base of the target.

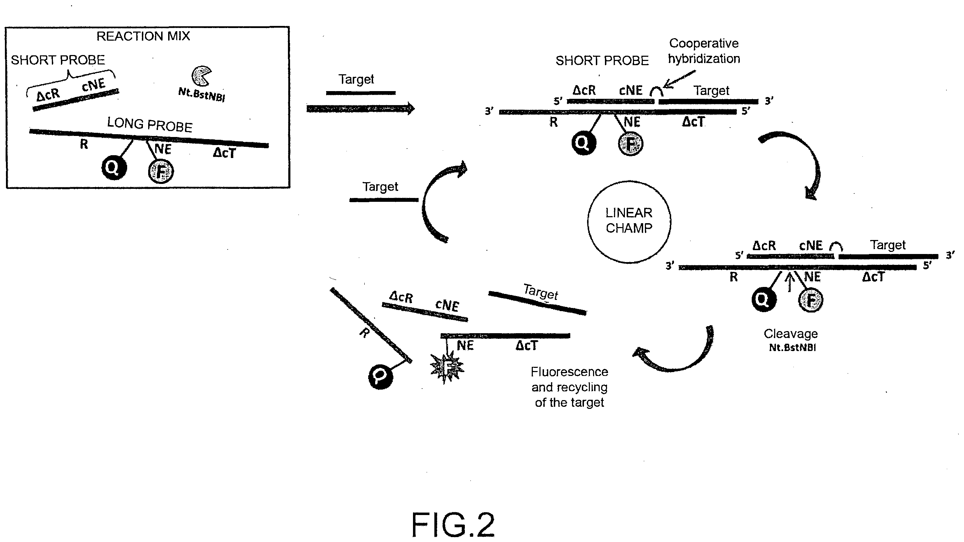

The cooperative hybridization of the three sequences (short probe, long probe and target) re-forms the functional double-stranded endonuclease recognition site, and therefore allows a strand of the long probe to be nicked by the endonuclease. In the presence of a nick, the reporter sequence R of the long probe spontaneously de-hybridizes from the short probe, because the complementary ΔcR portion is too short to maintain hybridization at the reaction temperature. As a result of the de-hybridization, the short probe also de-hybridizes, and this in turn destabilizes the hybridization of the target, leading to the release of the target in solution. The target molecule will thus be free to begin a new round of cooperative hybridization, nicking and release. Each round will release a reporter sequence in the solution. It should be noted that, because of the recycling, each target will release several copies of the reporter sequence, leading to signal amplification. This reporter sequence can be detected with any downstream detection methodology, for example through colorimetric detection strategies based on gold nanoparticles or in real time using a fluorescence de-quenching strategy, as shown in Figure 2. In this case, a fluorophore and a quencher are linked to two bases flanking the endonuclease recognition site, on the long probe. In a preferred embodiment, the quencher (Q) is linked to the 5'-end of the reporter sequence or in proximity thereto and the fluorophore (F) is linked to the 3'-end of the endonuclease recognition site or in proximity thereto. As long as the long probe is intact, the background fluorescence is very low because of the presence of the quencher. However, when the target in solution nicks the long probe, the fluorophore is separated from the quencher and the fluorescence signal increases. Each target hybridization event causes the release of a reporter sequence and the de-quenching of the fluorophore molecule.

In an alternative embodiment, the endonuclease may be a restriction endonuclease (which cuts the double strand) rather than a nicking endonuclease. In this case, the restriction endonuclease recognition site replaces the NE site shown in Figures 1 and 2, and the reaction is carried out at the required optimum temperature for the specific enzyme selected. In this embodiment, the sequences of all probes are designed so that their melting temperatures are suitable for the system at the reaction temperature of the enzyme.

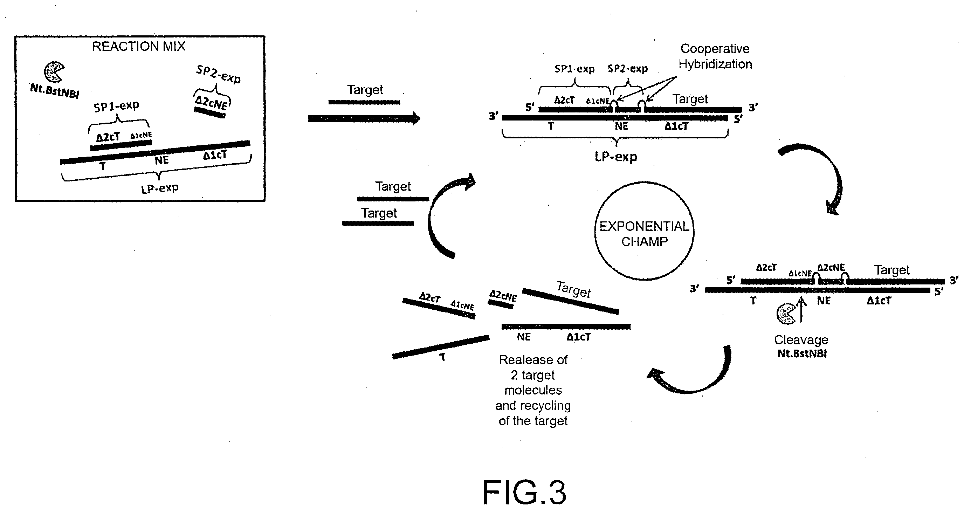

Figure 3 shows the exponential version of CHAMP which forms the object of the present invention. In this case, the long probe (LP-exp), comprises a 3' sequence identical to the target of interest (T) or, alternatively, a sequence identical to a portion of the target of interest including at least the portion complementary to Δ1cT, see below), a nicking endonuclease recognition site (NE) and a 5' sequence complementary to a first portion of the target of interest (Δ1cT). Also two different short probes are present in solution: a first short probe (SP1-exp) includes a 5' sequence complementary to a second portion of the target of interest (Δ2cT), and a 3' sequence complementary to a portion of the nicking endonuclease recognition site (Δ1cNE); the second short probe (SP2-exp) only includes a sequence that is complementary to the remaining portion of the nicking endonuclease recognition site (Δ2cNE).

Preferably, the Δ1cT sequence has a length comprised between 10 and 20 nucleotides.

Preferably, the Δ2cT sequence has a length comprised between 10 and 20 nucleotides.

Preferably, the Δ2cNE sequence has a length comprised between 0 and 5 nucleotides. In the case where Δ2cNE is not present, Δ1cNE would be complementary to the entire NE sequence and the system would include a single short probe, similarly to that of the linear CHAMP.

In the absence of the target, LP-exp and SP1-exp are hybridized to form a structure that is stable at the reaction temperature (for example 55°C), designated as "probe complex". In contrast, SP2-exp is free in solution because it is designed to be too short to hybridize at the reaction temperature.

Hybridization of SP2-exp and the target to the corresponding part of LP-exp becomes possible, thanks to the cooperative hybridization, similarly to what has been described above with reference to the linear CHAMP.

It should be noted that hybridization of the target alone, in the absence of SP2-exp, cannot occur.

Therefore, similarly to the linear version of CHAMP, the cooperative hybridization of the target and SP2-exp to the probe complex re-forms the intact endonuclease enzyme recognition site, allowing LP-exp to be enzymatically cleaved. The cleavage releases the T portion of LP-exp, which in turn destabilizes the binding of SP1-exp, causing its release. This in turn causes de-hybridization of both SP2-exp and the target.

Both the T portion of LP-Exp and the target, which have identical sequences, are free to begin a new round of cooperative hybridization, nicking and release. Therefore, in each round, two copies of the target are made available for the subsequent round. This results in an exponential amplification of the target.

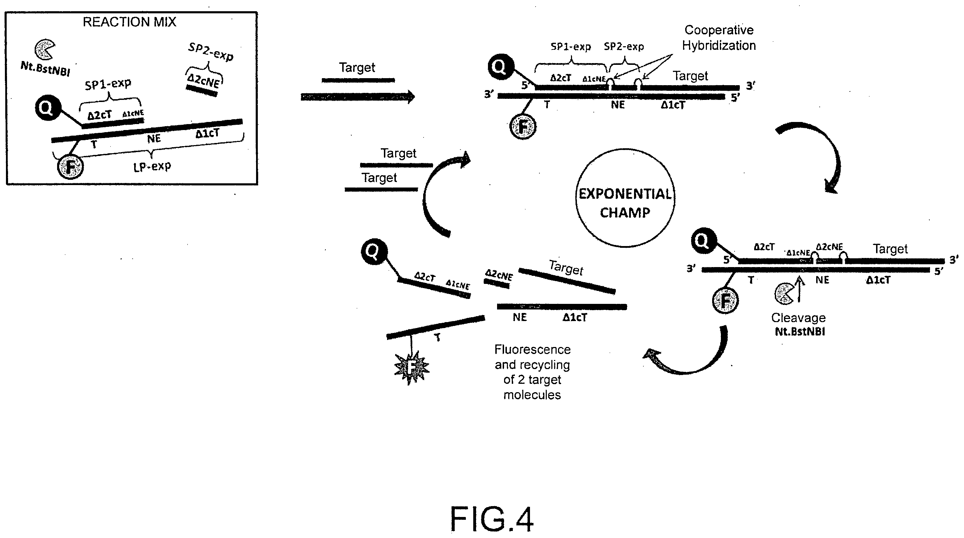

As for the linear version of CHAMP, also the exponential version can be coupled to a real-time fluorescence detection for quantitative analyses. In one embodiment provided by way of illustration, the fluorophore is linked to an internal base near the 3' end of the T portion of LP-exp, while the quencher is linked to the 5' end of SP1-exp. Thus, in the initial probe complex, the fluorophore and the quencher are very close to one another, as shown in Figure 4, and the fluorescence is quenched. Each target binding event causes nicking of LP-exp and the release of its T portion, which carries the fluorophore, thus leading to de-quenching of the fluorescence (increase in the fluorescence signal).

In an alternative embodiment, the endonuclease may be a restriction endonuclease (which cuts the double strand) rather than a nicking endonuclease. In this case, the restriction endonuclease recognition site replaces the NE site shown in Figures 3 and 4, and the reaction is carried out at the required optimum temperature for the specific enzyme selected. In this embodiment, the sequences of all probes are designed so that their melting temperatures are suitable for the system at the reaction temperature of the enzyme.

In all the embodiments described, both with reference to the linear CHAMP and with reference to the exponential CHAMP, a displacing probe can be added to the reaction mixture, to promote the recycling of the target and accelerate the reaction rate. The displacing probe competes with the target for binding to the long probe, after cleavage or nicking by the endonuclease.

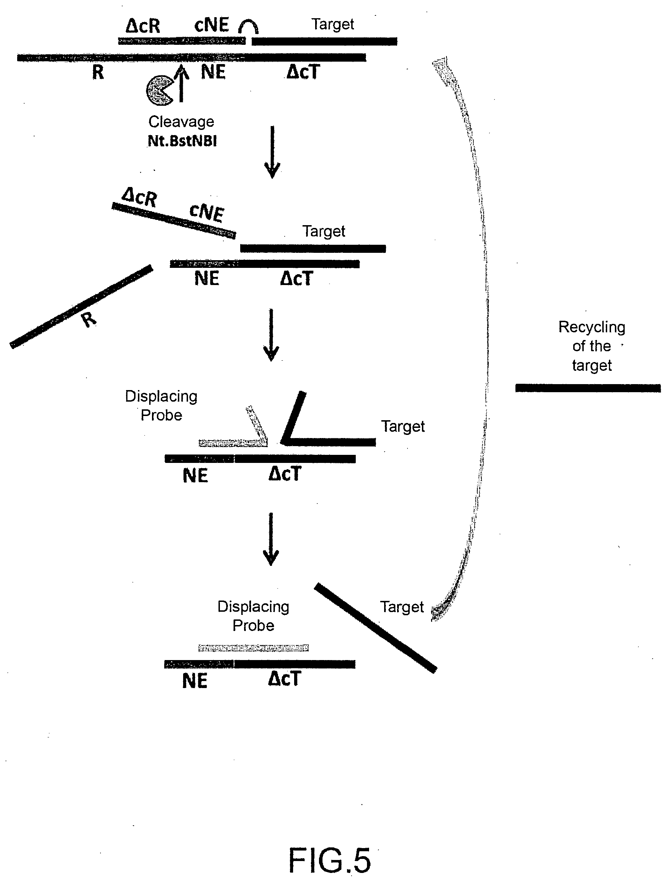

As illustrated in Figure 5 with reference to the linear CHAMP, the displacing probe is a single-stranded oligonucleotide which comprises:
- a 5' ΔcNE sequence complementary to a portion of the endonuclease recognition site, and
- a 3' ΔT sequence that comprises a portion of the target nucleic acid sequence.

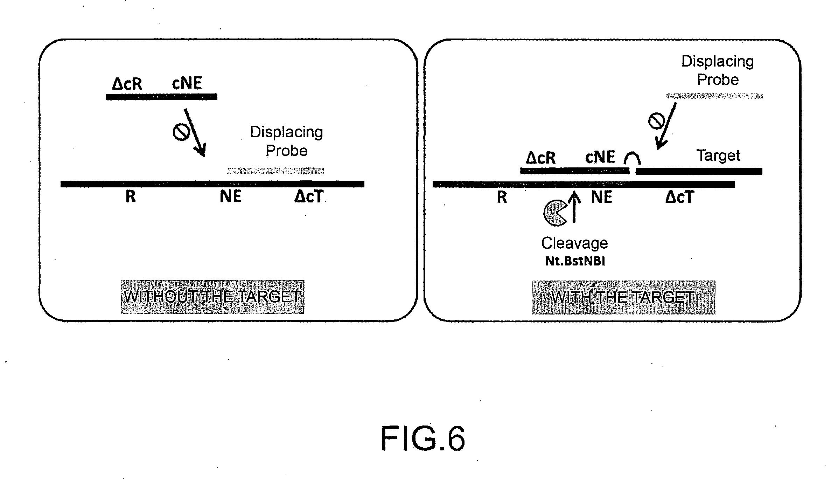

The displacing probe has a higher affinity for the fragment of the long probe than for the target, and displaces the target by toehold-mediated strand displacement. The displacing probe is present in the reaction mixture together with the target, even before the enzymatic cleavage or nicking. However, it does not prevent the target from binding to the intact long probe, since, when the intact long probe is present, the short probe and the target together compete with the displacing probe for binding to two regions on the long probe, which partially overlap the displacing probe binding site, preventing the binding of the displacing probe. It should be noted that in the absence of the target, the displacing probe also prevents the binding of the short probe alone (an unstable binding can occur in the absence of cooperative stabilization), thus preventing non-specific cleavage (independent from the target) of the long probe (Figure 6). In this way, the displacing probe has a dual function: to promote the recycling of the target, after cleavage; to prevent target-independent binding of the short probe, and thus prevent non-specific cleavage.

In an embodiment not shown, related to exponential CHAMP, the displacing probe is a single-stranded oligonucleotide which comprises:
- a 5' Δ3cNE sequence complementary to a portion of the endonuclease recognition site, and
- a 3' ΔT sequence that comprises a portion of the target nucleic acid sequence.

Both techniques for detection of nucleic acids which form the object of the present invention (linear CHAMP and exponential CHAMP) have the following advantages:
- simplicity of the reaction (one test tube, a single reaction step, one temperature, one enzyme), therefore they can be used with simple devices for field analysis and at the patient's point of care;
- they are based on cooperative hybridization, which does not require the design of complex probes, multiple primers or nucleic acids with secondary structures (hairpin); this feature, together with the simplicity of the reaction, allows the CHAMP to be used with any target nucleic acid;

- they can also be performed with RNA targets, without reverse transcription, which, for example, allows for direct analysis and miRNA quantification;
- they do not require a polymerase, which prevents non-specific amplification in the absence of the target from occurring;
- they can be coupled to real time fluorescent detection, which thus allows the target not only to be detected, but also to be quantified.

The examples that follow are provided for illustration purposes and should not be construed as limiting the scope of the present invention as defined in the appended claims.

### Example 1: miRNA21 detection

miRNA21 is overexpressed in many types of tumours, therefore the quantification of its expression level is relevant in tumour diagnostics.

miRNA21 was chosen as the target model for both the linear CHAMP and the exponential CHAMP.

For the linear version of CHAMP, the following synthetic sequences were used:
- LP = 5' ATCAGACTGATGTTGAGAGTCCTTTAGTGACAGAATAGA 3'
- SP = 5' ACTAAAGGACTC 3'
- Target (T) = 5' TCAACATCAGTCTGATAAGCTA 3'

The target sequence corresponds to the miRNA21 cDNA, as would be obtained by standard reverse transcription; however, the miRNA sequence can be used directly in the assay.

LP and SP, each at a final concentration of 500 nM, were mixed in solution with 15 U of the nicking enzyme Nt.BstNBI and with the enzyme reaction buffer, in a final volume of 50 µl.

For the exponential version of CHAMP, the following sequences were used:
- LP-exp = 5' TATCAGACTGATGTTGAGAGTCGGAGTCAACATCAGTCTGATAAGCTA 3'
- SP1-exp = 5' CAGACTGATGTTGACT 3'
- SP2-exp = 5' CCGACTC 3'
- Target (T) = 5' TCAACATCAGTCTGATAAGCTA 3'

A reaction mixture containing LP-exp at a final concentration of 1 µM and SP1-exp at a final concentration of 5 µM was prepared in Nt.BstNBI reaction buffer. This solution was heated to 90°C for 5 minutes, to destroy the secondary structure in LP-exp (i.e. a stable hairpin with a 16-bp stem), and then slowly cooled down to room temperature, to favour hybridization between LP-exp and SP1-exp and the formation of the probe complex. Then, 15 U of Nt.BstNBI nicking enzyme were added to the mixture in a final volume of 50 µl.

### Example 2: exemplary sequences for real-time fluorescence detection:

- LP = 5' ATCAGACTGATGTTGAGAGTCCTTTAGTGACAGAATAGA 3' (Real-Time Linear CHAMP)
- LP-exp = 5' TATCAGACTGATGTTGAGAGTCGGAGTCAACATCAGTCTGATAAGCTA 3' (Real-Time Exponential CHAMP)
- SP1-exp = 5' CAGACTGATGTTGACT 3' (Real-Time Exponential CHAMP)

In LP, the positions of the fluorophore and of the quencher are underlined with a single and a double underscore, respectively.

In LP-exp, the position of the fluorophore is underlined with a single underscore. In SP1-exp, the position of the quencher is underlined with a double underscore.

### Example 3: Detection of a genomic DNA target

The genomic DNA is double-stranded and therefore, before the CHAMP, a single-stranded target must be generated. This preliminary step can be carried out easily with various strategies and is a necessary step also for most of the isothermal amplification schemes of the prior art, and therefore does not represent a limitation to the broad applicability of CHAMP. For instance, an ssDNA target can be generated in the same reaction tube, selecting a region of the genome containing two sites for the endonuclease employed, for example Nt.BstNBI. For example, the plasmid pNL4.3, which contains the HIV-1 virus genome, contains the sequence **GAGTC**GAAGGTGTCCTTTTGCGCC**GAGTC** (the Nt.BstNBI sites are underlined), which generates a 24-mer ssDNA, which is a suitable substrate for CHAMP.

## Claims

1. A kit for detecting a target nucleic acid sequence (T), the kit comprising:
i) an endonuclease enzyme;
ii) a long probe (LP) consisting of a single-stranded oligonucleotide consisting of:
- a 3' reporter sequence (R),
- an endonuclease recognition site (NE), and
- a 5' ΔcT sequence complementary to a portion of the target nucleic acid sequence to be detected; and
iii) a short probe (SP) consisting of a single-stranded oligonucleotide consisting of:
- a 5' ΔcR sequence complementary to a portion of the reporter sequence, and
- a 3' cNE sequence complementary to the endonuclease recognition site,
wherein both the 5' ΔcT sequence and the 5' ΔcR sequence are sufficiently short so that hybridization of the short probe to the long probe does not occur in the absence of the target nucleic acid sequence, and wherein the kit does non comprise a polymerase enzyme.

2. The kit according to claim 1, wherein the 5' ΔcT sequence is from 10 to 20 nucleotides in length and the 5' ΔcR sequence is from 0 to 10 nucleotides in length.

3. The kit according to claim 1 or 2, wherein the reporter sequence is labelled with a quencher (Q) and the endonuclease recognition site is labelled with a fluorophore (F).

4. The kit according to claim 3, wherein the quencher is located at or in proximity of the 5'-end of the reporter sequence and the fluorophore is located at or in proximity of the 3'-end of the endonuclease recognition site.

5. The kit according to any of claims 1 to 4, further comprising a displacing probe (DP) which is a single-stranded oligonucleotide comprising:
- a 5' ΔcNE sequence complementary to a portion of the endonuclease recognition site, and
- a 3' ΔT sequence comprising a portion of the target nucleic acid sequence.

6. The kit according to any of claims 1 to 5, wherein the endonuclease is selected from a nicking endonuclease and a restriction endonuclease.

7. A method of detecting a target nucleic acid sequence (T) in a nucleic acid sample, the method comprising the steps of:
a) adding to the nucleic acid sample a reaction mixture comprising:
i) an endonuclease enzyme;
ii) an excess of a long probe (LP) consisting of a single-stranded oligonucleotide consisting of:
- a 3' reporter sequence (R),
- an endonuclease recognition site (NE), and
- a 5' ΔcT sequence complementary to a portion of the target nucleic acid sequence to be detected; and
iii) an excess of a short probe (SP) consisting of a single-stranded oligonucleotide consisting of:
- a 5' ΔcR sequence complementary to a portion of the reporter sequence, and
- a 3' cNE sequence complementary to the restriction enzyme recognition site,
wherein both the 5' ΔcT sequence and the 5' ΔcR sequence are sufficiently short so that hybridization of the short probe to the long probe does not occur in the absence of the target nucleic acid sequence, and
wherein the reaction mixture does not contain a polymerase enzyme;
b) allowing the reaction mixture to react with the nucleic acid sample at a temperature at which the endonuclease is active, whereby, if the target nucleic acid sequence is present in the nucleic acid sample, a double-stranded endonuclease enzyme recognition site is formed by cooperative hybridization of the short probe and the target nucleic acid sequence to the long probe, and the double-stranded endonuclease enzyme recognition site is then nicked or cleaved by the endonuclease, whereby a free reporter sequence is released from the long probe; and
c) detecting the free reporter sequence as an indication of the presence of the target nucleic acid sequence in the nucleic acid sample.

8. The method according to claim 7, wherein the temperature at which the reaction mixture is allowed to react with the nucleic acid sample in step b) is comprised between 50°C and 70°C, preferably is about 55°C.

9. The method according to claim 7 or 8, wherein the 5' ΔcT sequence is from 10 to 20 nucleotides in length and the 5' ΔcR sequence is from 0 to 10 nucleotides in length.

10. The method according to any of claims 7 to 9, wherein the free reporter sequence is detected by detecting a change in a fluorescence parameter, preferably a decrease or increase in fluorescence intensity.

11. The method according to any of claims 7 to 10, wherein the reaction mixture further comprises a displacing probe (DP) which is a single-stranded oligonucleotide comprising:
- a 5' ΔcNE sequence complementary to a portion of the endonuclease recognition site, and
- a 3' ΔT sequence comprising a portion of the target nucleic acid sequence.

12. The method according to any of claims 7 to 11, wherein the endonuclease enzyme is selected from a nicking endonuclease and a restriction endonuclease.

## Patentansprüche

1. Set zum Ermitteln einer Zielnukleinsäuresequenz (T), wobei das Set umfasst:
i) ein Endonukleaseenzym;
ii) eine lange Sonde (LP) bestehend aus einem einsträngigen Oligonukleotid bestehend aus:
- einer 3'-Reportersequenz (R),
- einer Endonukleaseerkennungsstelle (NE), und
- einer 5'-ΔcT-Sequenz, die komplementär ist zu einem Abschnitt der zu ermittelnden Zielnukleinsäuresequenz; und
iii) einer kurzen Sonde (SP) bestehend aus einem einsträngigen Oligonukleotid bestehend aus:
- einer 5'-AcR-Sequenz, die komplementär zu einem Abschnitt der Reportersequenz ist, und
- einer 3'-cNE-Sequenz, die komplementär zu einer Endonukleaseerkennungsstelle ist,
wobei sowohl die 5'-ΔcT-Sequenz und die 5'-ΔcR-Sequenz ausreichend kurz sind, so dass eine Hybridisierung der kurzen Sonde zu der langen Sonde in der Abwesenheit der Zielnukleinsäuresequenz nicht auftritt, und wobei das Set kein Polymeraseenzym umfasst.

2. Set nach Anspruch 1, wobei die 5'-ΔcT-Sequenz in der Länge aus 10 bis 20 Nukleotiden besteht, und die 5'-ΔcR-Sequenz in der Länge aus 0 bis 10 Nukleotiden besteht.

3. Set nach Anspruch 1 oder 2, wobei die Reportersequenz mit einem Quencher (Q) markiert ist, und die Endonukleaseerkennungsstelle mit einem Fluorophor (F) markiert ist.

4. Set nach Anspruch 3, wobei der Quencher an oder in der Nähe des 5'-Endes der Reportersequenz angeordnet ist, und das Fluorophor an oder in der Nähe des 3'-Endes der Endonukleaseerkennungsstelle angeordnet ist.

5. Set nach einem der Ansprüche 1 bis 4, ferner umfassend eine Versetzungssonde (DP), welche ein einsträngiges Oligonukleotid ist, welches umfasst:
- eine 5'-ΔcNE-Sequenz, die komplementär ist zu einem Abschnitt der Endonukleaseerkennungsstelle, und
- eine 3'-ΔT-Sequenz, umfassend einen Abschnitt der Zielnukleinsäuresequenz.

6. Set nach einem der Ansprüche 1 bis 5, wobei die Endonuklease ausgewählt ist aus einer schneidenden Endonuklease und einer Restriktionsendonuklease.

7. Verfahren zum Ermitteln einer Zielnukleinsäuresequenz (T) in einer Nukleinsäureprobe, wobei das Verfahren die Schritte umfasst:
a) Hinzufügen einer Reaktionsmischung zu der Nukleinsäureprobe, welche umfasst:
i) ein Endonukleaseenzym;
ii) einen Überschuss einer langen Sonde (LP) bestehend aus einem einsträngigen Oligonukleotid bestehend aus:
- einer 3'-Reportersequenz (R),
- einer Endonukleaseerkennungsstelle (NE), und
- einer 5'-ΔcT-Sequenz, die komplementär ist zu einem Abschnitt der zu ermittelnden Zielnukleinsäuresequenz; und
iii) einem Überschuss einer kurzen Sonde (SP) bestehend aus einem einsträngigen Oligonukleotid bestehend aus:
- einer 5'-ΔcR-Sequenz, die komplementär zu einem Abschnitt der Reportersequenz ist, und
- einer 3'-cNE-Sequenz, die komplementär zu der Endonukleaseerkennungsstelle ist,
wobei sowohl die 5'-ΔcT-Sequenz und die 5'-ΔcR-Sequenz ausreichend kurz sind, so dass eine Hybridisierung der kurzen Sonde zu der langen Sonde in der Abwesenheit der Zielnukleinsäuresequenz nicht auftritt, und
wobei die Reaktionsmischung kein Polymeraseenzym enthält;
b) Erlauben der Reaktionsmischung mit der Nukleinsäureprobe bei einer Temperatur zu reagieren, bei welcher die Endonuklease aktiv ist, wodurch, falls die Zielnukleinsäuresequenz in der Nukleinsäureprobe vorliegt, eine doppelsträngige Endonukleaseenzymerkennungsstelle durch kooperative Hybridisierung der kurzen Sonde und der Zielnukleinsäuresequenz zu der langen Sonde ausgebildet wird, und die doppelsträngige Endonukleaseenzymerkennungsstelle dann durch die Endonuklease geschnitten oder gespalten wird, wodurch eine freie Reportersequenz durch die lange Sonde freigesetzt wird; und
c) Ermitteln der freien Reportersequenz als ein Hinweis für das Vorliegen der Zielnukleinsäuresequenz in der Nukleinsäureprobe.

8. Verfahren nach Anspruch 7, wobei die Temperatur, bei der es der Reaktionsmischung erlaubt wird mit der Nukleinsäureprobe in Schritt b) zu reagieren, zwischen 50 °C und 70 °C liegt, bevorzugt etwa 55 °C beträgt.

9. Verfahren nach Anspruch 7 oder 8, wobei die 5'-ΔcT-Sequenz in der Länge aus 10 bis 20 Nukleotiden besteht, und die 5'-ΔcR-Sequenz in der Länge aus 0 bis 10 Nukleotiden besteht.

10. Verfahren nach einem der Ansprüche 7 bis 9, wobei die freie Reportersequenz durch Ermitteln einer Änderung in einem Fluroreszenzparameter ermittelt wird, bevorzugt einer Abnahme oder einem Anstieg in der Fluoreszenzintensität.

11. Verfahren nach einem der Ansprüche 7 bis 10, wobei die Reaktionsmischung ferner eine Versetzungssonde (DP) umfasst, welche ein einsträngiges Oligonukleotid ist, welches umfasst:
- eine 5'-cNE-Sequenz, die komplementär ist zu einem Abschnitt der Endonukleaseerkennungsstelle, und
- eine 3'-ΔT-Sequenz, umfassend einen Abschnitt der Zielnukleinsäuresequenz.

12. Verfahren nach einem der Ansprüche 7 bis 11, wobei das Endonukleaseenzym ausgewählt ist aus einer schneidenden Endonuklease und einer Restriktionsendonuklease.

## Revendications

1. Trousse pour détecter une séquence d'acide nucléique cible (T), la trousse comprenant :
i) une enzyme endonucléase ;
ii) une sonde longue (LP) consistant en un oligonucléotide simple brin consistant en :
- une séquence de rapporteur (R) en 3',
- un site de reconnaissance d'endonucléase (NE), et
- une séquence ΔcT en 5' complémentaire à une partie de la séquence d'acide nucléique cible à détecter ; et
iii) une sonde courte (SP) consistant en un oligonucléotide simple brin consistant en :
- une séquence ΔcR en 5' complémentaire à une partie de la séquence de rapporteur, et
- une séquence cNE en 3' complémentaire au site de reconnaissance d'endonucléase,
dans laquelle à la fois la séquence ΔcT en 5' et la séquence ΔcR en 5' sont suffisamment courtes pour qu'une hybridation de la sonde courte à la sonde longue ne se produise pas en l'absence de la séquence d'acide nucléique cible, et dans laquelle la trousse ne comprend pas d'enzyme polymérase.

2. Trousse selon la revendication 1, dans laquelle la séquence ΔcT en 5' est d'une longueur de 10 à 20 nucléotides et la séquence ΔcR en 5' est d'une longueur de 0 à 10 nucléotides.

3. Trousse selon la revendication 1 ou 2, dans laquelle la séquence de rapporteur est marquée avec un extincteur (Q) et le site de reconnaissance d'endonucléase est marqué avec un fluorophore (F).

4. Trousse selon la revendication 3, dans laquelle l'extincteur est situé au niveau ou à proximité de l'extrémité 5' de la séquence de rapporteur et le fluorophore est situé au niveau ou à proximité de l'extrémité 3' du site de reconnaissance d'endonucléase.

5. Trousse selon l'une quelconque des revendications 1 à 4, comprenant en outre une sonde de déplacement (DP) qui est un oligonucléotide simple brin comprenant :
- une séquence ΔcNE en 5' complémentaire à une partie du site de reconnaissance d'endonucléase, et
- une séquence ΔT en 3' comprenant une partie de la séquence d'acide nucléique cible.

6. Trousse selon l'une quelconque des revendications 1 à 5, dans laquelle l'endonucléase est sélectionnée parmi une endonucléase de coupure et une endonucléase de restriction.

7. Procédé de détection d'une séquence d'acide nucléique cible (T) dans un échantillon d'acide nucléique, le procédé comprenant les étapes suivantes :
a) l'ajout à l'échantillon d'acide nucléique d'un mélange réactionnel comprenant :
i) une enzyme endonucléase ;
ii) un excès d'une sonde longue (LP) consistant en un oligonucléotide simple brin consistant en :
- une séquence de rapporteur (R) en 3',
- un site de reconnaissance d'endonucléase (NE), et
- une séquence ΔcT en 5' complémentaire à une partie de la séquence d'acide nucléique cible à détecter ; et
iii) un excès d'une sonde courte (SP) consistant en un oligonucléotide simple brin consistant en :
- une séquence ΔcR en 5' complémentaire à une partie de la séquence de rapporteur, et
- une séquence cNE en 3' complémentaire au site de reconnaissance d'enzyme de restriction,
dans lequel à la fois la séquence ΔcT en 5' et la séquence ΔcR en 5' sont suffisamment courtes pour qu'une hybridation de la sonde courte à la sonde longue ne se produise pas en l'absence de la séquence d'acide nucléique cible, et
dans lequel le mélange réactionnel ne contient pas d'enzyme polymérase ;
b) le fait de permettre au mélange réactionnel de réagir avec l'échantillon d'acide nucléique à une température à laquelle l'endonucléase est active, selon lequel, si la séquence d'acide nucléique cible est présente dans l'échantillon d'acide nucléique, un site de reconnaissance d'enzyme endonucléase double brin est formé par hybridation coopérative de la sonde courte et de la séquence d'acide nucléique cible à la sonde longue, et le site de reconnaissance d'enzyme endonucléase double brin est ensuite coupé ou clivé par l'endonucléase, selon lequel une séquence de rapporteur libre est libérée à partir de la sonde longue ; et
c) la détection de la séquence de rapporteur libre en tant qu'indication de la présence de la séquence d'acide nucléique cible dans l'échantillon d'acide nucléique.

8. Procédé selon la revendication 7, dans lequel la température à laquelle le mélange réactionnel est laissé à réagir avec l'échantillon d'acide nucléique à l'étape b) est comprise entre 50 °C et 70 °C, de préférence est d'environ 55 °C.

9. Procédé selon la revendication 7 ou 8, dans lequel la séquence ΔcT en 5' est d'une longueur de 10 à 20 nucléotides et la séquence ΔcR en 5' est d'une longueur de 0 à 10 nucléotides.

10. Procédé selon l'une quelconque des revendications 7 à 9, dans lequel la séquence de rapporteur libre est détectée par la détection d'un changement d'un paramètre de fluorescence, de préférence une diminution ou une augmentation d'intensité de fluorescence.

11. Procédé selon l'une quelconque des revendications 7 à 10, dans lequel le mélange réactionnel comprend en outre une sonde de déplacement (DP) qui est un oligonucléotide simple brin comprenant :
- une séquence ΔcNE en 5' complémentaire à une partie du site de reconnaissance d'endonucléase, et
- une séquence ΔT en 3' comprenant une partie de la séquence d'acide nucléique cible.

12. Procédé selon l'une quelconque des revendications 7 à 11, dans lequel l'enzyme endonucléase est sélectionnée parmi une endonucléase de coupure et une endonucléase de restriction.


### SEQUENCE LISTING

<110> FONDAZIONE ISTITUTO ITALIANO DI TECNOLOGIA (IIT)
<120> A method and a kit for nucleic acid detection
<130> I0183813-EC
<160> 10
<170> PatentIn version 3.5
<210> 1
   <211> 39
   <212> DNA
   <213> artificial sequence
<220>
   <223> Long probe (LP)
<400> 1
   atcagactga tgttgagagt cctttagtga cagaataga 39
<210> 2
   <211> 12
   <212> DNA
   <213> artificial sequence
<220>
   <223> Short probe (SP)
<400> 2
   actaaaggac tc 12
<210> 3
   <211> 22
   <212> DNA
   <213> artificial sequence
<220>
   <223> Target (T)
<400> 3
   tcaacatcag tctgataagc ta 22
<210> 4
   <211> 48
   <212> DNA
   <213> artificial sequence
<220>
   <223> Long probe-exponential (LP-exp)
<400> 4
   tatcagactg atgttgagag tcggagtcaa catcagtctg ataagcta 48
<210> 5
   <211> 16
   <212> DNA
   <213> artificial sequence
<220>
   <223> First short probe-exponential (SP1-exp)
<400> 5
   cagactgatg ttgact 16
<210> 6
   <211> 22
   <212> DNA
   <213> artificial sequence
<220>
   <223> Target (T)
<400> 6
   tcaacatcag tctgataagc ta 22
<210> 7
   <211> 39
   <212> DNA
   <213> artificial sequence
<220>
   <223> Long probe (LP)
<400> 7
   atcagactga tgttgagagt cctttagtga cagaataga 39
<210> 8
   <211> 48
   <212> DNA
   <213> artificial sequence
<220>
   <223> Long probe-exponential (LP-exp)
<400> 8
   tatcagactg atgttgagag tcggagtcaa catcagtctg ataagcta 48
<210> 9
   <211> 16
   <212> DNA
   <213> artificial sequence
<220>
   <223> Firs short probe-exponential (SP1-exp)
<400> 9
   cagactgatg ttgact 16
<210> 10
   <211> 29
   <212> DNA
   <213> artificial sequence
<220>
   <223> HVI-1 sequence
<400> 10
   gagtcgaagg tgtccttttg cgccgagtc 29